(19) Europäisches Patentamt

European Patent Office

Office européen des brevets

(11) Numéro de publication: **0 481 899 B1**

(12) **FASCICULE DE BREVET EUROPEEN**

(45) Date de publication de fascicule du brevet: **28.09.94** (51) Int. Cl.⁵: **A23L 2/38**, A23L 1/30

(21) Numéro de dépôt: **91402789.1**

(22) Date de dépôt: **18.10.91**

Le dossier contient des informations techniques présentées postérieurement au dépôt de la demande et ne figurant pas dans le présent fascicule.

(54) **Boisson diététique destinée à permettre de soutenir l'effort.**

(30) Priorité: **18.10.90 FR 9012902**

(43) Date de publication de la demande:
**22.04.92 Bulletin 92/17**

(45) Mention de la délivrance du brevet:
**28.09.94 Bulletin 94/39**

(84) Etats contractants désignés:
**AT BE CH DE DK ES FR GB GR IT LI LU NL SE**

(56) Documents cités:
**WO-A-89/05101**
**US-A- 3 894 148**

**PATENT ABSTRACTS OF JAPAN vol. 9, no. 78 (C-274)(1801) 6Avril 1985 & JP-A-59 210 870 ( TAIYOU KAGAKU KK ) 29 Novembre 1984**

**WORLD PATENTS INDEX LATEST Week 8217, Derwent Publications**

**Ltd., London, GB; AN 82-33891E (17) & JP-A-57 047 446 (NIPPON OILS & FATS KK) 18 Mars 1982**

**Lebensmittelllexikon, 1981 page 259 VEB Leipzig**

(73) Titulaire: **PERNOD-RICARD**
**142, Boulevard Haussmann**
**F-75008 Paris (FR)**

(72) Inventeur: **Guezennec, Yannick**
**30 rue Guérin**
**F-77300 Fontainebleau (FR)**
Inventeur: **Koziet, Joseph**
**83, rue Beauregard**
**F-94350 Villiers sur Marne (FR)**
Inventeur: **Thiry, Michel**
**28 rue de la Longueraie**
**F-91270 Vigneux sur Seine (FR)**

(74) Mandataire: **Ahner, Francis**
**CABINET REGIMBEAU**
**26, avenue Kléber**
**F-75116 Paris (FR)**

**Description**

La présente invention se rapporte à des compositions destinées à être absorbées avant et pendant l'effort, de façon à permettre de soutenir cet effort en limitant les effets dus à l'épuisement des réserves disponibles de l'organisme. Après l'effort cette composition peut aider à restaurer les réserves de glycogène.

On sait que le muscle en activité doit transformer de l'énergie chimique, l'adénosine triphosphate (ATP) en énergie mécanique, mais les réserves d'ATP cellulaire sont minimes. Il existe donc différentes sources d'énergie pour renouveler cet ATP cellulaire.

La voie de l'anaérobiose lactique met en jeu la phosphocréatinine (CP) disponible immédiatement dans le muscle selon le schéma

CP + ADP⇌ATP + créatinine, et permet des exercices intenses et brefs.

La voie de la glycolyse anaérobie utilise le glucose sanguin et le glycogène musculaire, mais provoque la formation d'acide lactique, et se trouve donc limitée par la capacité de l'organisme à utiliser les lactates. Elle permet des efforts physiques à moyen terme.

Enfin, les efforts de longue durée sollicitent des processus aérobies, qui passent par l'oxydation complète du glucose issu de la glycogénolyse et de la néoglucogénèse puis l'oxydation des acides gras libres issus de l'hydrolyse des graisses corporelles qui prennent le relais pour la fourniture d'énergie.

Il existe une consommation critique d'oxygène, spécifique d'un individu et de son entraînement, au-delà de laquelle l'organisme se fatigue. Cette VO$_2$ max est améliorée par l'entraînement.

Normalement, un sujet adulte utilise environ 2400 calories par jour, soit 100 calories/heure. Lors d'un effort important, cette consommation peut passer à 1200 calories/heure, ce qui équivaut à 60 molécules d'ATP.

L'organisme risque alors d'être sujet à des défaillances, dont les causes les plus fréquentes sont l'hypoglycémie, l'épuisement du glycogène musculaire et la déshydratation.

On s'est donc attaché à fournir des compléments nutritionnels destinés à éviter la survenue de ces défaillances, qui limitent les performances et sont néfastes pour la santé de l'athlète.

Des produits diététipres sont connus, voir par exemple JP-A-59 210 870.

La plupart des produits diététiques de l'effort sont à prédominance glucidique sous forme de glucose, afin de répondre aux besoins glucidiques immédiats de l'effort musculaire. Toutefois ces produits, surtout lorsqu'il s'agit de boissons devant être absorbées pendant un effort au long cours, peuvent entraîner des troubles digestifs très gênants.

Par ailleurs, l'absorption de ce type de produit en début d'exercice entraîne une sécrétion d'insuline par l'organisme qui peut provoquer une hypoglycémie réactionnelle. Par la suite l'apport de glucose s'oppose à la lipolyse endogène et donc à l'utilisation des acides gras libres par le muscle, qui sont une source d'énergie importante au cours d'un effort de longue durée.

Afin de pallier à ces inconvénients, la Demanderesse à découvert de manière tout à fait surprenante qu'une composition contenant essentiellement une association de glucides et de lipides donnait des résultats nettement améliorés, mis en évidence notamment par la mesure de différents paramètres sanguins.

C'est pourquoi la présente invention se rapporte à une composition diététique destinée à être absorbée avant, pendant, ou après un effort, caractérisée en ce qu'elle comporte des glucides et des lipides, lesdits lipides constituant 10 à 55 % du contenu calorique de la composition.

Outre les glucides et les lipides, la composition pourra bien évidemment contenir tous les adjuvants nécessaires à sa formulation tels que conservateurs, agents de sapidité, de coloration, épaississants, gélifiants, etc.

Par effort, on entend la mise en jeu volontaire des muscles, par des phénomènes physiologiques et psychologiques, tendant à provoquer une activité supérieure à la normale d'un groupe musculaire ou de l'organisme entier. Cet effort nécessite l'augmentation du débit cardiaque, du rythme respiratoire et entraîne celle des besoins énergétiques.

La composition selon l'invention permet de soutenir un effort en limitant la survenue de la fatigue et du surmenage provoqués par l'inadéquation entre les apports nutritionnels et les besoins énergétiques.

L'apport énergétique de ladite composition sera constitué au moins à 10 %, et jusqu'à 55 % du total des calories par des lipides. Une autre partie de l'apport énergétique se fera sous forme de glucides.

La présente invention concerne une composition diététique d'effort, dans laquelle tout ou partie des constituants lipidiques, qui sont dans les proportions définies plus haut, consiste en des triglycérides à chaîne moyenne. Ces triglycérides à chaîne moyenne résultent de l'estérification du glycérol par des acides gras en C$_6$ à C$_{10}$, saturés ou non.

Plus précisément, une composition diététique selon l'invention peut comporter des constituants lipidiques à raison de 10 à 55 % de l'apport calorique total, consistant en triglycérides à chaîne moyenne et également en triglycérides à chaîne longue.

De préférence, les triglycérides à chaîne moyenne représentent au moins 40 % des lipides totaux.

De manière également préférée, les triglycérides à chaîne longue contiennent au moins 30 % d'acides gras polyinsaturés. On évitera cependant d'utiliser des huiles de poisson dans les compositions selon l'invention.

Les glucides représentent de préférence de 30 à 90 % du contenu calorique de la composition.

Selon un aspect de l'invention, la composition diététique telle qu'elle a été définie comprend, en tant que constituant glucidique, du fructose et du glucose.

Plus particulièrement, la présente invention a pour objet une composition diététique d'effort comportant des glucides et des lipides dans les proportions définies plus haut et dans laquelle le fructose représente au moins 40 % des glucides totaux.

Des résultats particulièrement favorables sont obtenus pour des compositions selon l'invention dans lesquelles le rapport des concentrations glucides/ lipides est supérieur ou égal à 1. De façon préférée, le rapport fructose/lipides dans les compositions selon la présente invention est supérieur ou égal à 0,5.

En effet, des études réalisées à l'aide de substrats marqués au carbone 13 ont montré que le fructose, ainsi que les triglycérides à chaîne moyenne (en particulier à 8 atomes de carbone) sont oxydés rapidement par l'organisme humain pendant l'exercice physique en gaz carbonique, apportant ainsi une énergie utilisable directement pour l'exercice physique et qui se rapproche de celle apportée par du glucose ou de l'amidon.

Les acides gras à longue chaîne ne sont oxydés que lentement.

Par contre, si on compare la réponse insulinémique des 4 substrats énergétiques envisagés, on constate que seuls le glucose et l'amidon entraînent vraiment une forte insulinémie alors que le fructose et les triglycérides n'ont pas cet inconvénient.

En outre, on peut mesurer chez des sportifs pendant les phases d'effort et de récupération, différents paramètres sanguins qui témoignent du potentiel énergétique et de la souffrance de l'organisme. On mesure ainsi les acides gras libres, les corps cétoniques, le glucose , le lactate et le glycérol, au cours d'une épreuve sportive, chez les individus ayant absorbé une boisson selon l'invention : les taux plasmatiques révèlent une utilisation plus efficace des substrats énergétiques apportés sous forme de lipides et une meilleure mobilisation des graisses de réserve par rapport à ceux observés avec des compositions traditionnelles à base de glucides.

Selon l'un de ses aspects, la présente invention concerne des compositions destinées à être absorbées pendant l'effort, et contenant, dans le produit prêt à absorber, du glucose à une concentration comprise entre environ 10 et 35 g/l, du fructose à une concentration comprise entre environ 10 et 35 g/l, des triglycérides à chaîne longue à une concentration comprise entre environ 1 et 10 g/l et des triglycérides à chaîne moyenne à une concentration comprise entre environ 1 et 10 g/l.

Selon un mode de réalisation préféré, la présente invention concerne une composition, caractérisée en ce qu'elle contient 10 g/l de glucose, du fructose à une concentration de 10 g/l, des triglycérides à chaîne longue à une concentration de 5 g/l et des triglycérides à chaîne moyenne à une concentration de 5 g/l.

Dans un autre mode de réalisation, la composition selon la présente invention contient 20 g/l de glucose, 20 g/l de fructose, 2,5 g/l de triglycérides à chaîne longue et 2,5 g/l de triglycérides à chaîne moyenne.

Selon un de ses aspects préférés, la composition selon l'invention se présente sous forme liquide.

Cette présentation permet, outre l'apport énergétique optimisé qui est déterminé par les compositions selon l'invention, de compenser les pertes hydriques survenant lors d'un effort musculaire, par la sudation destinée à lutter contre la tendance à l'élévation de la température corporelle (par exemple, le débit sudoral d'un marathonien est de 1 à 2 litres/heure).

Ces compositions auront donc le double avantage de répondre aux besoins énergétiques de l'organisme et de prévenir la déshydratation par ingestion avant, pendant et/ou après l'effort.

En outre ces compositions présentent une très bonne tolérance digestive, contrairement à ce que l'on peut observer avec des boissons glucidiques, d'autant plus celles ayant un fort pouvoir osmolaire.

La composition selon l'invention peut se présenter sous forme concentrée ou sèche, en vue de sa dilution ultérieure. Les concentrations finales dans le produit prêt à boire pourront varier en fonction des conditions de l'épreuve sportive et les seuils de concentration dépendent de la dilution effectuée par le consommateur du produit.

C'est ainsi que la présente invention concerne une composition diététique d'effort, liquide dont l'apport calorique est constitué de 10 à 55% sous forme de lipides telle qu'elle a pu être définie plus haut, et qui est absorbée à raison de 2 à 2,5 l pendant un effort, par exemple pendant un effort de 2 à 3 heures ; ceci sans provoquer de gastralgies.

Selon un autre aspect de l'invention, la composition peut se présenter sous forme de tablettes, pâtes ou biscuits, destinés à être absorbés avant, pendant ou après l'effort, éventuellement redilués avec de l'eau par l'utilisateur.

Les exemples qui suivent sont destinés à illustrer l'invention sans aucunement en limiter la portée.

Dans ces exemples, on se référera aux figures suivantes :

**FIGURE 1 :** Evolution des taux d'oxydation des substrats marqués au $^{13}$C : glucose, triglycérides à chaîne longue (TCL), triglycérides à chaîne moyenne (TCC) à deux doses 25 et 50 g per os.

**FIGURE 2 :** Evolution des taux d'oxydation des substrats marqués au $^{13}$C : glucose, fructose, amidon.

**FIGURE 3 :** Protocole expérimental observé lors du déroulement des triathlons.

**FIGURE 4 :** Evolution du taux de glycérol plasmatique chez des sujets ayant absorbé respectivement les boissons A et B.

**FIGURE 5 :** Evolution des taux d'acides gras libres plasmatiques chez des sujets ayant absorbé respectivement les boissons A et B.

## EXEMPLE 1 : ETUDE DU METABOLISME DE DIFFERENTS SUBSTRATS MARQUES AU $^{13}$C.

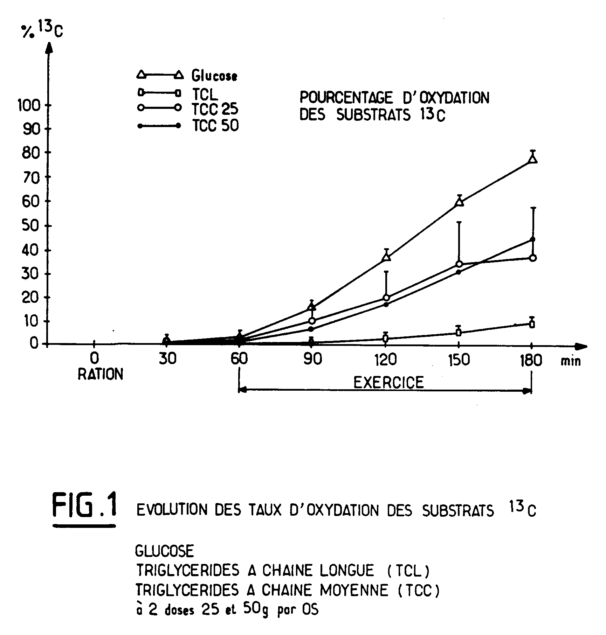

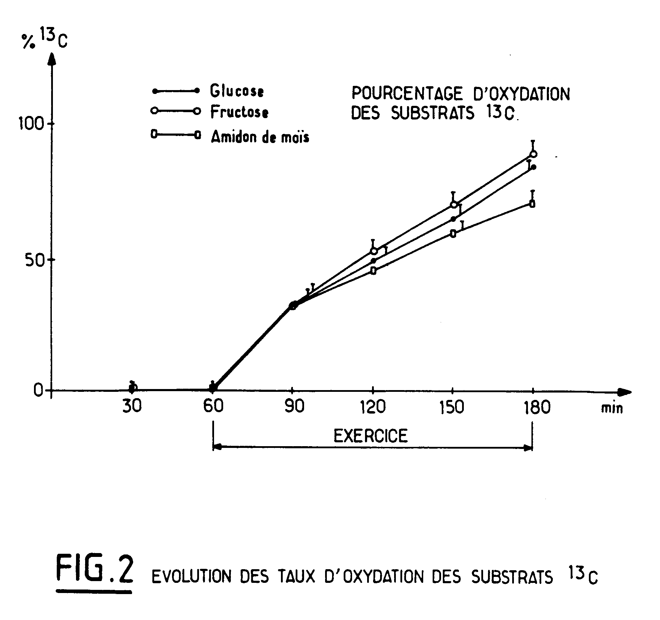

Les figures 1 et 2 permettent de comparer l'utilisation de différents substrats carbonés comme source d'énergie.

Cette utilisation est suivie par l'évolution des taux plasmatiques de substrats oxydés.

On met ainsi en évidence que le fructose et les triglycérides à chaîne moyenne fournissent une énergie rapidement utilisable pour l'exercice physique, comparable à celle fournie par du glucose ou de l'amidon.

Les acides gras à chaîne longue ne sont oxydés que lentement, et génèrent une énergie à plus long terme.

Les triglycérides et le fructose présentent en outre l'avantage de ne pas entraîner d'insulinémie réactionnelle, à la différence de ce qui est observé avec le glucose ou l'amidon.

## EXEMPLE 2 : COMPARAISON DES PERFORMANCES D'UNE BOISSON D'EFFORT TRADITIONNELLE ET D'UNE BOISSON SELON L'INVENTION.

On a fourni à des sportifs entraînés, lors d'épreuve de triathlon (1500 m natation - 40 km vélo - 10 km course) soit une boisson glucidique traditionnelle (A) soit une boisson de l'effort associant glucides et lipides (B).

Composition de deux boissons

**A**

| SUBSTRAT | CONCENTRATION | VALEUR ENERGETIQUE (Kcal) |
|---|---|---|
| GLUCOSE ET MALTODEXTINE ) ) | ) 76 g/l ) | 304 |

**B**

| SUBSTRAT | CONCENTRATION | VALEUR ENERGETIQUE (Kcal) |
|---|---|---|
| GLUCOSE | 10g/l | 40 |
| FRUCTOSE | 10g/l | 40 |
| TRIGLYCERIDES | | |
| chaîne moyenne | 5g/l | 35 |
| chaîne longue | 5g/l | 45 |
| | | ----- |
| | | 160 |

Les paramètres métaboliques ont été mesurés à l'issue de chaque épreuve de chaque triathlon par prise de sang.

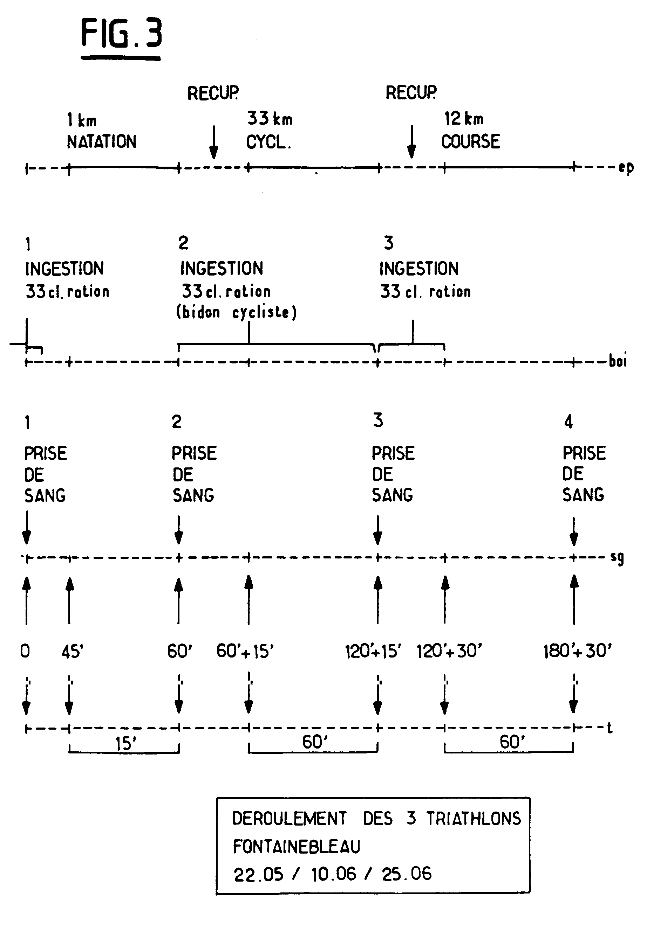

La figure 3 indique le protocole expérimental.

Les paramètres sanguins mesurés ont été :

- acides gras libres, corps cétoniques,

- glucose lactate glycérol.

Les résultats ont été analysés selon 2 techniques statistiques :
- analyse de variance,
- analyse en composante principale.

Les deux techniques aboutissent à la même conclusion.

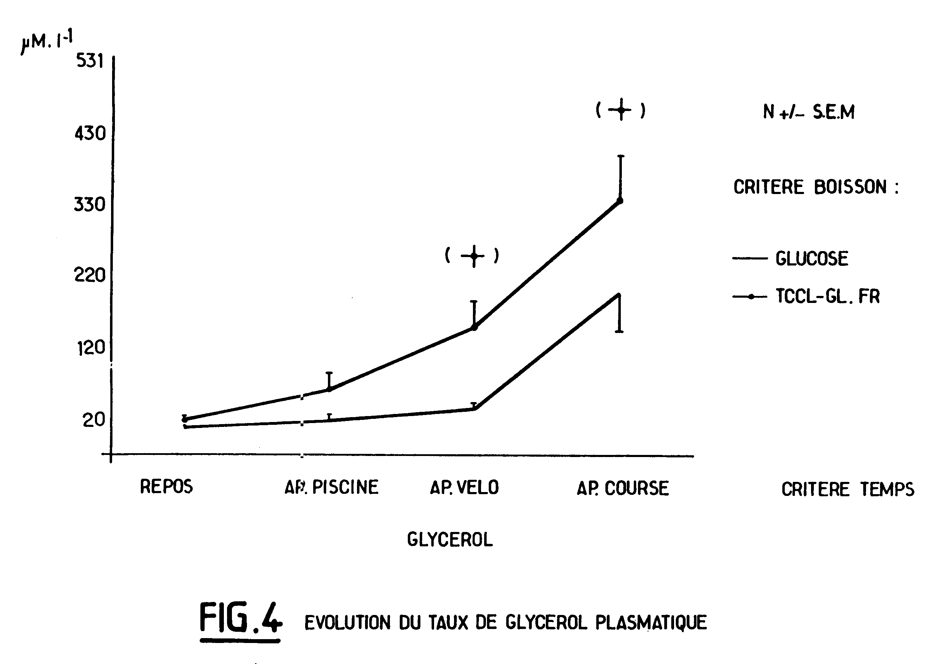

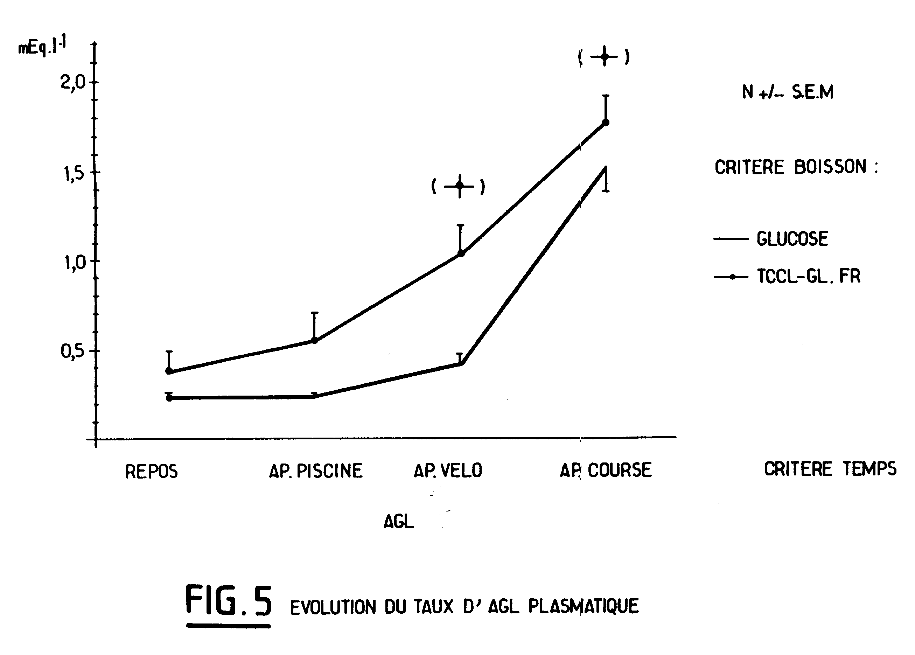

La boisson B fait apparaître au cours des épreuves un taux plus élevé de glycérol et d'acides gras libres plasmatiques (cf figure 4 et 5.)

Ce résultat est important. En effet la consommation à intervalles réguliers d'une boisson ne contenant que des glucides inhibe la lipolyse endogène. Par contre la boisson contenant les triglycérides favorise la lipolyse qui fournit des acides gras libres et du glycérol, auxquels s'ajoutent les produits d'hydrolyse des triglycérides. Les acides gras libres sont ainsi disponibles plus tôt pour être oxydés par le muscle. En outre le glycérol participe au maintien de la glycémie par néoglucogénèse.

D'autre part, on a observé que la boisson B n'entraînait aucun trouble digestif, alors qu'il est connu que lors d'une épreuve de longue durée, 30 % des sujets présentent des troubles digestifs qui sont d'autant plus intenses que la boisson à un fort pouvoir osmolaire.

Une épreuve de course à pied de 100 km a confirmé la bonne tolérence gastrique de la boisson B dont la consommation a été de 2,0 l à 2,5 l. Aucun des 5 coureurs n'a souffert de gastralgies.

## Revendications

1. Composition diététique destinée à être absorbée avant, pendant ou après un effort, caractérisée en ce qu'elle comporte des glucides et des lipides, lesdits lipides constituant 10 à 55% du contenu calorique de la composition et consistant en partie en triglycérides à chaîne moyenne ($C_6$ à $C_{10}$), et en ce que les glucides sont constitués en partie par du fructose.

2. Composition selon la revendication 1, caractérisée en ce que les triglycérides à chaîne moyenne représentent au moins 40% des lipides totaux.

3. Composition selon l'une des revendications 1 et 2, caractérisée en ce que les triglycérides à chaîne moyenne sont formés à partir d'acides gras en $C_6$ à $C_{10}$.

4. Composition selon l'une des revendications 1 à 3, caractérisée en ce que le fructose représente au moins 40% des glucides totaux.

5. Composition selon l'une des revendications 1 à 3, caractérisée en ce que le rapport des concentrations fructose/lipides est supérieur ou égal à 0,5.

6. Composition selon l'une des revendications 1 à 5, caractérisée en ce que les lipides contiennent également des triglycérides à chaîne longue.

7. Composition selon la revendication 6, caractérisée en ce que les triglycérides à chaîne longue comprennent au moins 30% d'acides gras polyinsaturés.

8. Composition selon l'une des revendications 1 à 7, caractérisée en ce qu'elle contient sous forme prête à absorber du glucose à une concentration comprise entre environ 10 et 35 g/l, du fructose à une concentration comprise entre environ 10 et 35 g/l, des triglycérides à chaîne longue à une concentration comprise entre environ 1 et 10 g/l et des triglycérides à chaîne moyenne à une concentration comprise entre environ 1 et 10 g/l.

9. Composition selon l'une des revendications 1 à 8, caractérisée en ce qu'elle contient 20 g/l de glucose, du fructose à une concentration de 20 g/l, des triglycérides à chaîne longue à une concentration de 2,5 g/l et des triglycérides à chaîne moyenne à une concentration de 2,5 g/l.

10. Composition selon l'une des revendications 1 à 9, caractérisée en ce qu'elle est liquide.

11. Composition selon la revendication 10, caractérisée en ce qu'elle est absorbée à raison de 2 à 2,5 l pendant un effort de 2 à 3 heures.

**Claims**

1.  Dietary composition intended to be absorbed before, during or after an activity, characterized in that it comprises carbohydrates and lipids, said lipids constituting 10 to 55% of the calorie content of the composition and consisting partially of ($C_6$ to $C_{10}$) medium chain triglycerides, and in that the carbohydrates partially consist of fructose.

2.  Composition according to Claim 1, characterized in that the medium chain triglycerides represent at least 40% of the total lipids.

3.  Composition according to either of Claims 1 and 2, characterized in that the medium chain triglycerides are produced from $C_6$ to $C_{10}$ fatty acids.

4.  Composition according to one of Claims 1 to 3, characterized in that the fructose represents at least 40% of the total carbohydrates.

5.  Composition according to one of Claims 1 to 3, characterized in that the fructose/lipid concentration ratio is greater than or equal to 0.5.

6.  Composition according to one of Claims 1 to 5 characterized in that the lipids also contain long chain triglycerides.

7.  Composition according to Claim 6, characterized in that the long chain triglycerides contain at least 30% of polyunsaturated fatty acids.

8.  Composition according to one of Claims 1 to 7, characterized in that it contains, in a form ready to be absorbed, glucose at a concentration of between about 10 and 35 g/l, fructose at a concentration of between about 10 and 35 g/l, long chain triglycerides at a concentration of between about 1 and 10 g/l and medium chain triglycerides at a concentration of between about 1 and 10 g/l.

9.  Composition according to one of Claims 1 to 8, characterized in that it contains 20 g/l of glucose, fructose at a concentration of 20 g/l, long chain triglycerides at a concentration of 2.5 g/l and medium chain triglycerides at a concentration of 2.5 g/l.

10. Composition according to one of Claims 1 to 9, characterized in that it is liquid.

11. Composition according to Claim 10, characterized in that it is absorbed in an amount of 2 to 2.5 l during an activity of 2 to 3 h.

**Patentansprüche**

1.  Diätetische Zusammensetzung für die Einnahme vor, während oder nach einer Beanspruchung (Anstrengung), dadurch gekennzeichnet, daß sie Kohlenhydrate und Lipide enthält, wobei die Lipide 10 bis 55 % des Energieinhalts der Zusammensetzung ausmachen und zum Teil bestehen aus Triglyceriden mit mittlerer Kettenlänge ($C_6$-$C_{10}$) und daß die Kohlenhydrate zum Teil aus Fructose bestehen.

2.  Zusammensetzung nach Anspruch 1, dadurch gekennzeichnet, daß die Triglyceride mit mittlerer Kettenlänge mindestens 40 % der Gesamtlipide ausmachen.

3.  Zusammensetzung nach einem der Ansprüche 1 und 2, dadurch gekennzeichnet, daß die Triglyceride mit mittlerer Kettenlänge aus $C_6$-$C_{10}$-Fettsäuren hergestellt wurden.

4.  Zusammensetzung nach einem der Ansprüche 1 bis 3, dadurch gekennzeichnet, daß die Fructose mindestens 40 % der Gesamt-Kohlenhydrate ausmacht.

5.  Zusammensetzung nach einem der Ansprüche 1 bis 3, dadurch gekennzeichnet, daß das Verhältnis zwischen den Konzentrationen an Fructose und Lipiden gleich oder höher ist als 0,5.

6. Zusammensetzung nach einem der Ansprüche 1 bis 5, dadurch gekennzeichnet, daß die Lipide auch Triglyceride mit langer Kettenlänge enthalten.

7. Zusammensetzung nach Anspruch 6, dadurch gekennzeichnet, daß die Triglyceride mit langer Kettenlänge mindestens 30 % mehrfach ungesättigte Fettsäuren enthalten.

8. Zusammensetzung nach einem der Ansprüche 1 bis 7, dadurch gekennzeichnet, daß sie in einer gebrauchsfertigen Form enthält Glucose in einer Konzentration zwischen etwa 10 und 35 g/l, Fructose in einer Konzentration zwischen etwa 10 und 35 g/l, Triglyceride mit langer Kettenlänge in einer Konzentration zwischen etwa 1 und 10 g/l und Triglyceride mit mittlerer Kettenlänge in einer Konzentration zwischen etwa 1 und 10 g/l.

9. Zusammensetzung nach einem der Ansprüche 1 bis 8, dadurch gekennzeichnet, daß sie enthält 20 g/l Glucose, Fructose in einer Konzentration von 20 g/l, Triglyceride mit langer Kettenlänge in einer Konzentration von 2,5 g/l und Triglyceride mit einer mittleren Kettenlänge in einer Konzentration von 2,5 g/l.

10. Zusammensetzung nach einem der Ansprüche 1 bis 9, dadurch gekennzeichnet, daß sie flüssig ist.

11. Zusammensetzung nach Anspruch 10, dadurch gekennzeichnet, daß sie während einer Beanspruchung (Anstrengung) von 2 bis 3 h in einer Menge von 2 bis 2,5 l eingenommen wird.

FIG.1 EVOLUTION DES TAUX D'OXYDATION DES SUBSTRATS $^{13}$C

GLUCOSE
TRIGLYCERIDES A CHAINE LONGUE (TCL)
TRIGLYCERIDES A CHAINE MOYENNE (TCC)
à 2 doses 25 et 50g par OS

FIG.2 EVOLUTION DES TAUX D'OXYDATION DES SUBSTRATS $^{13}$C

# FIG.3

DEROULEMENT DES 3 TRIATHLONS
FONTAINEBLEAU
22.05 / 10.06 / 25.06

FIG.4 EVOLUTION DU TAUX DE GLYCEROL PLASMATIQUE

FIG.5 EVOLUTION DU TAUX D' AGL PLASMATIQUE